(19) Europäisches Patentamt / European Patent Office / Office européen des brevets

(11) **EP 1 915 089 B1**

(12) **EUROPEAN PATENT SPECIFICATION**

(45) Date of publication and mention
of the grant of the patent:
**10.04.2013 Bulletin 2013/15**

(21) Application number: **06780158.9**

(22) Date of filing: **21.07.2006**

(51) Int Cl.:
***A61B 5/053*** (2006.01)

(86) International application number:
**PCT/IB2006/052501**

(87) International publication number:
**WO 2007/017779 (15.02.2007 Gazette 2007/07)**

(54) **ELECTRIC IMPEDANCE IMAGING SYSTEM, METHOD, AND COMPUTER PROGRAM**

ELEKTROIMPEDANZ-BILDGEBUNGSSYSTEM, VERFAHREN UND COMPUTERPROGRAMM

SYSTEME D'IMAGERIE A IMPEDANCE ELECTRIQUE, PROCÉDÉ, ET PROGRAMME D'ORDINATEUR

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.08.2005 EP 05107280**

(43) Date of publication of application:
**30.04.2008 Bulletin 2008/18**

(73) Proprietors:
• **Koninklijke Philips Electronics N.V.**
**5621 BA Eindhoven (NL)**
Designated Contracting States:
**AT BE BG CH CY CZ DK EE ES FI FR GB GR HU IE IS IT LI LT LU LV MC NL PL PT RO SE SI SK TR**
• **Philips Intellectual Property & Standards GmbH**
**20099 Hamburg (DE)**
Designated Contracting States:
**DE**

(72) Inventors:
• **KATSCHER, Ulrich**
**NL-5656 AA Eindhoven (NL)**
• **FINDEKLEE, Christian**
**NL-5656 AA Eindhoven (NL)**

• **VERNICKEL, Peter**
**NL-5656 AA Eindhoven (NL)**

(74) Representative: **Verweij, Petronella Danielle et al**
**Philips Intellectual Property & Standards**
**P.O. Box 220**
**5600 AE Eindhoven (NL)**

(56) References cited:
**US-A1- 2005 052 182**

• **MUFTULER L TUGAN ET AL: "Resolution and contrast in magnetic resonance electrical impedance tomography (MREIT) and its application to cancer imaging." TECHNOLOGY IN CANCER RESEARCH & TREATMENT DEC 2004, vol. 3, no. 6, December 2004 (2004-12), pages 599-609, XP002435672 ISSN: 1533-0346**
• **SHAI LEVY ET AL: "Electromagnetic Impedance Tomography (EMIT):A New Method for Impedance Imaging" IEEE TRANSACTIONS ON MEDICAL IMAGING, IEEE SERVICE CENTER, PISCATAWAY, NJ, US, vol. 21, no. 6, June 2002 (2002-06), XP011076308 ISSN: 0278-0062**

**Description**

The invention pertains to an electric impedance imaging system to explore the electrical conductivity and permittivity distribution of an object, and a method and a computer program.

Such an electric impedance imaging system is known from the US patent application US2003/0160622.

This known electric impedance imaging system concerns an apparatus that utilizes an array of RF probes to measure along multiple signal channels the non-resonant thermal noise, which is produced in a sample. From this measurement the non-resonant thermal noise correlation is derived. The detected noise correlation is a function of the spatial overlap of the electro-magnetic fields of the RF probes and the spatial distribution of the conductivity of the sample, i.e. an object to be examined. In fact, the noise correlation between the respective channels (j and k) is equal

to $\int \sigma(r) \underline{E}_j(r) \underline{E}_k^*(r) \mathrm{d}V$ , where $\underline{E}_i$ is the electric field strength phasor for channel i and $\sigma$ is the electrical conductivity distribution. The electrical conductivity distribution is obtained by way of an inverse problem approach that involves a matrix-inversion or back-projection.

The known electric impedance imaging system has the function of electrical impedance tomography (EIT). The EIT techniques involves generating an electrical current distribution in the object, either through direct contact or inductive coupling and then measuring this distribution through an array of detectors designed to measure either current or voltage. These measurements are then used to calculate an impedance map.

Alternative approaches are mentioned in the paper 'Resolution and constast in magnetic resonance electrical impedance tomography (MREIT) and its application to cancer imaging' in Technol. cancer Res. Treat 3(2004)599-609 by L.T. Muftuler et al. and in the paper 'Electromagnetic impedance tomography (EMIT): a new method form impedance imaging' in IEEE Trasn.Med.Imaging 21(2002)676-687. These approached concern electric impedance tomography on the basis of the response to the injection of an electrical current in the object to be examined.

An aim of the present invention is to provide an electric impedance imaging system which relative to the known electric impedance imaging system has an improved spatial resolution for the electrical conductivity and permittivity distribution.

This aim is achieved by the electric impedance imaging system of the invention to explore an object's electrical conductivity and permittivity distribution according to claim 1.

The electric impedance imaging system is capable for imaging both the electrical conductivity and permittivity. These quantities may alternatively be considered as the real and imaginary parts of a complex electrical impedance. The electric impedance imaging system according to the invention derives the magnetic induction field strength distribution from the magnetic resonance signals. These magnetic resonance signals are acquired with a very good spatial resolution when magnetic field gradient encoding is applied. An RF receive antennae system is employed to acquire the magnetic resonance signals. Such magnetic field gradient encoding schemes are known per se in the field of magnetic resonance imaging. The electric impedance imaging system of the invention is provided with a signal acquisition system of a modem magnetic resonance imaging system. In this way sub-millimeter resolution for the magnetic induction field strength distribution that is derived from the magnetic resonance signals is achieved.

Additionally, the electrical conductivity and permittivity distribution is obtained in a more direct way as compared to how the known electric impedance imaging system operates. Notably, no inverse problem needs to be solved and accordingly there does not occur a problem related to the inverse problem being ill-posed. The known electrical impedance tomography is prone to problems being ill-posed since the spatial frequencies of the electric field distributions are generally much lower than the spatial frequencies of the electrical impedance distribution.

Furthermore, the computational effort that the electric impedance imaging system of the invention requires is less, because the electric impedance imaging system of the invention requires differentiation that is mathematically less complex than the matrix inversion or back-projection that the known electric impedance imaging system uses. Notably, the aspect of ill-position is avoided. Further, the electrical impedance imaging system is able to efficiently handle a large amount of data. Notably, according to the invention the computation of the electrical conductivity and permittivity is made on a pixel-by-pixel basis. The electric impedance imaging system is for example employed to explore the electrical permittivity distribution of a patient to be examined. Notably, different biological tissues appear to have different values of the electrical permittivity, so that different tissues can be distinguished on the basis of their values of the electrical permittivity.

These and other aspects will be further elaborated with reference to the embodiments defined in the dependent claims.

An RF transmit antennae system is employed to transmit the electro-magnetic excitation field. Often, the RF transmit antennae system and the RF receive antennae systems, respectively, make use of the same hardware components that are operated in a receive and a transmit mode, respectively. The magnetic resonance signal level that is measured due to the electro-magnetic excitation depends on the circular polarisation components of magnetic induction field strength having excitation polarisation orientation and receive polarisation orientation, respectively. More in particular, the signal level of the magnetic resonance signals is about proportional to the receive circular polarised component of the magnetic induction field strength. Further, the signal level of the magnetic resonance signals is about proportional to a goniometric function of the excitation circular polarised component of the magnetic induction field strength. In particular, it appears that the goniometric function is the sine function, since at zero flip angle, zero signal is obtained.

The respective excitation and receive circular components of the magnetic induction field strength can be accurately obtained from magnetic resonance signal acquisitions at respective different flip angles associated with the electro-magnetic excitation field applied in the individual series of acquisitions. Namely, the electro-magnetic excitation field causes a precession of spins in the object around an axis along the direction of a main magnetic field that is applied by the electric impedance imaging system. The precession occurs in that the spins rotate at an angle, i.e. the flip angle around the direction of the main magnetic field. The respective circularly polarised components of the magnetic induction field strength are derived on the basis of fitting the signal levels of the acquired magnetic resonance signals to the goniometric function dependence.

An accurate result for the electric field strength distribution is obtained in an iterative approach. This iterative approach is initialised on the basis of a first estimate of the electric field strength distribution that is made on the basis of (i) the geometry of the RF transmit antennae system that is employed to generate the excitation electro-magnetic field, (ii) the electric voltages applied to various elements (e.g. coils) of the RF transmit antennae system, (iii) the size and shape of the object to be examined and (iv) a coarse estimate of the electrical conductivity and permittivity of the object to be examined. On the basis of this initialisation there is computed a better, more accurate, estimate of the electrical conductivity and permittivity distribution. This update is then again employed as an input, replacing the previous coarser estimate of the electrical conductivity and permittivity distribution. On the basis of the updated distribution the computation is re-iterated to obtain successive electrical conductivity and permittivity distribution that are increasingly accurately representing the actual electrical distribution of the object to be examined. The first estimate of the distribution is for example taken as a single mean value or is taken as literature values for respective compartments of the object to be examined.

According to another aspect one of the polarisation components of the magnetic induction field is derived from the electric field strength distribution, notably as obtained from the iterative estimate. Then the received magnetic resonance signals from a receive-only RF receive antenna can be analysed to derive the electrical impedance distribution, without the need to include control over the polarisation component of the magnetic induction field of the excitation electro-magnetic field. Notably, this allows to perform the excitation with a quadrature body-coil (QBC) of a magnetic resonance imaging system and acquire the magnetic resonance signals by means of a receive-only surface coil.

According to a further aspect the RF transmit/receive antenna system can use a switchable polarisation orientation of the emitted electromagnetic field. In this embodiment the RF transmit/receive is operated in the transmit mode in the excitation polarisation orientation and is operated in the receive mode in the receive polarisation orientation. The excitation polarisation and the receive polarisation are opposite circular polarisations. From measurements at the respective polarisation orientation the respective circularly polarised components of the magnetic induction field strength are derived which are then employed together with the electric field strength distribution to compute the complex electrical permittivity. An alternative, slightly more cumbersome way to effectively perform measurements at respective polarisation orientations is to perform respective measurements in which the orientation is changed patient to be examined.

In a further aspect several RF receive antennae are employed that have complementary spatial sensitivity profiles. This enables to avoids spatial regions where the total electrical field strength is close to zero. This avoids that the computation of the electrical permittivity distribution requires division by small numbers, which would lead to numerical instability.

According to another aspect, an image, e.g. in the form of a volumetric dataset or a tomographic, cross-sectional tomographic image is reconstructed from the electrical permittivity distribution. Thus an image that represents the electrical permittivity distribution is formed that shows differences in e.g. tissue type that are represented by the differences in electrical permittivity value. Thus, differences between tissue types that are not distinguished by conventional diagnostic imaging modalities, such as magnetic resonance imaging, x-ray imaging, ultra-sound imaging, nuclear medicine etc. are now seen as different by the electric impedance imaging system of the invention.

The invention also relates to a method of electrical impedance imaging. The method of electrical impedance imaging of the invention is defined in the independent method claim. Because the method of electric impedance imaging of the invention employs the magnetic resonance signals to derive the electrical permittivity distribution of the object to be examined, a good (i.e. sub-millimetre) spatial resolution of the electrical permittivity distribution is achieved. It is noted that the method of the invention is conveniently operated by way of a computer program.

Further, the invention relates to a computer program for electric impedance imaging. The computer program of the invention is defined in the independent computer programme claim. The computer program of the invention can be installed in a processor, e.g. in the processor of a magnetic resonance imaging system. Then, the magnetic resonance imaging system having the computer program of the invention installed is enabled to perform the method of electric impedance imaging of the invention. The computer program of the invention may be supplied on a data carrier such as a CD-rom disk. Alternatively, the computer program of the invention may be downloaded from a data network, such as the world-wide web. In particular, the computer programme of the invention may be installed in the processor of a magnetic resonance imaging system to enable the magnetic resonance imaging system to perform electrical impedance imaging. Also, different versions or updates of the computer programme of the invention may easily be installed to replace a previous version without the need for substantial adaptation of the system hardware, such as RF excitation and/or RF receiver antennae (coils).

These and other aspects of the invention will be elucidated with reference to the embodiments described hereinafter and with reference to the accompanying drawing wherein

Figure 1 shows diagrammatically an electric impedance imaging system in the form of a magnetic resonance imaging system that is adapted to carry out the function of electrical impedance imaging;
Figure 2 shows a flow chart for Electrical Conductivity Imaging;

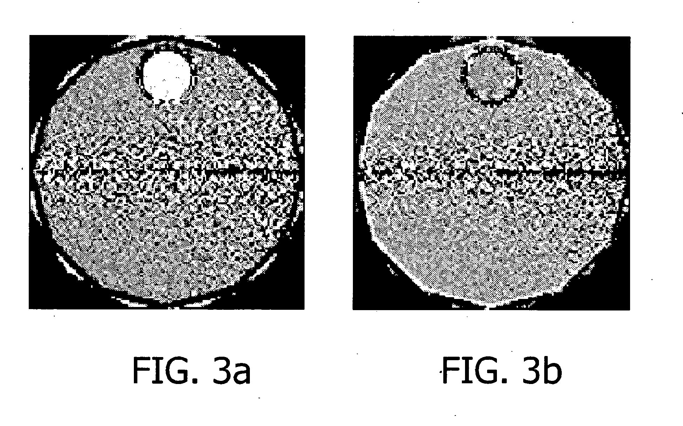

Figure 3 shows the results of a demonstration of the basic numerical feasibility of the approach of the invention.

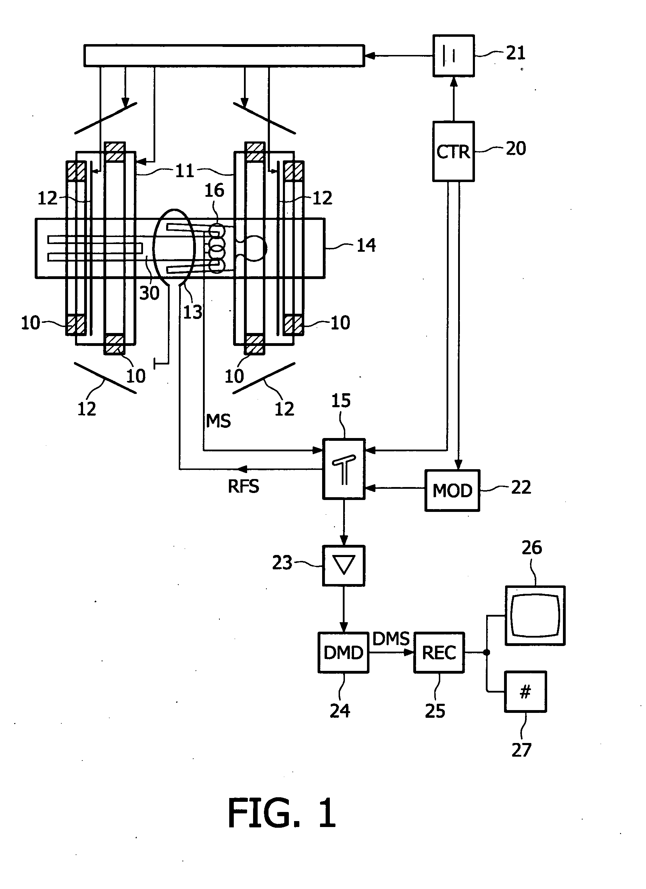

Figure 1 shows diagrammatically an electric impedance imaging system in the form of a magnetic resonance imaging system that is adapted to carry out the function of electrical impedance imaging. The magnetic resonance imaging system includes a set of main coils 10 whereby the steady, uniform magnetic field is generated. The main coils are constructed, for example in such a manner that they enclose a tunnel-shaped examination space. The patient to be examined is placed on a patient carrier which is slid into this tunnel-shaped examination space. The magnetic resonance imaging system also includes a number of gradient coils 11, 12 whereby magnetic fields exhibiting spatial variations, notably in the form of temporary gradients in individual directions, are generated so as to be superposed on the uniform magnetic field. The gradient coils 11, 12 are connected to a controllable power supply unit 21. The gradient coils 11, 12 are energised by application of an electric current by means of the power supply unit 21; to this end the power supply unit is fitted with electronic gradient amplification circuit that applies the electric current to the gradient coils so as to generate gradient pulses (also termed 'gradient waveforms') of appropriate temporal shape. The strength, direction and duration of the gradients are controlled by control of the power supply unit. The magnetic resonance imaging system also includes transmission and receiving coils 13, 16 for generating the RF excitation pulses and for picking up the magnetic resonance signals, respectively. The transmission coil 13 is preferably constructed as a body coil 13 whereby (a part of) the object to be examined can be enclosed. The body coil is usually arranged in the magnetic resonance imaging system in such a manner that the patient 30 to be examined is enclosed by the body coil 13 when he or she is arranged in the magnetic resonance imaging system. The body coil 13 acts as a transmission antenna for the transmission of the RF excitation pulses and RF refocusing pulses. Preferably, the body coil 13 involves a spatially uniform intensity distribution of the transmitted RF pulses (RFS). The same coil or antenna is usually used alternately as the transmission coil and the receiving coil. Furthermore, the transmission and receiving coil is usually shaped as a coil, but other geometries where the transmission and receiving coil acts as a transmission and receiving antenna for RF electromagnetic signals are also feasible. The transmission and receiving coil 13 is connected to an electronic transmission and receiving circuit 15.

It is to be noted that it is alternatively possible to use separate receiving and/or transmission coils 16. For example, surface coils 16 can be used as receiving and/or transmission coils. Such surface coils have a high sensitivity in a comparatively small volume. The receiving coils, such as the surface coils, are connected to a demodulator 24 and the received magnetic resonance signals (MS) are demodulated by means of the demodulator 24. The demodulated magnetic resonance signals (DMS) are applied to a reconstruction unit. The receiving coil is connected to a preamplifier 23. The preamplifier 23 amplifies the RF resonance signal (MS) received by the receiving coil 16 and the amplified RF resonance signal is applied to a demodulator 24. The demodulator 24 demodulates the amplified RF resonance signal. The demodulated resonance signal contains the actual information concerning the local spin densities in the part of the object to be imaged. Furthermore, the transmission and receiving circuit 15 is connected to a modulator 22. The modulator 22 and the transmission and receiving circuit 15 activate the transmission coil 13 so as to transmit the RF excitation and refocusing pulses. The reconstruction unit derives one or more image signals from the demodulated magnetic resonance signals (DMS), which image signals represent the image information of the imaged part of the object to be examined. The reconstruction unit 25 in practice is constructed preferably as a digital image processing unit 25 which is programmed so as to derive from the demodulated magnetic resonance signals the image signals which represent the image information of the part of the object to be imaged. The signal on the output of the reconstruction monitor 26, so that the monitor can display the magnetic resonance image. It is alternatively possible to store the signal from the reconstruction unit 25 in a buffer unit 27 while awaiting further processing.

The magnetic resonance imaging system according to the invention is also provided with a control unit 20, for example in the form of a computer which includes a (micro)processor. The control unit 20 controls the execution of the RF excitations and the application of the temporary gradient fields. To this end, the computer program according to the invention is loaded, for example, into the control unit 20 and the reconstruction unit 25. According to the present invention the control unit and the reconstruction unit are arranged to control the magnetic resonance imaging system to operate as an electric impedance imaging system. Notably, the RF excitation is performed with a circular excitation polarisation, the magnetic resonance signals are acquired at the circular receive polarisation that is e.g. opposite the excitation polarisation. Further, the reconstruction unit is arranged, e.g. programmed to compute the electric field strength distribution, derive magnetic induction field strength distribution from the acquired magnetic resonance signals and compute the electrical permittivity distribution. Further, of course, the reconstructor is also arranged to reconstruct volumetric datasets or tomographic cross sectional images from the electrical permittivity distribution.

In the electric impedance imaging system of the invention, the measurement instead of the noise correlations between different coil measurements is employed of the magnetic induction field (H field) of the coils involved. The measured H field might have the same spatial resolution as standard MR images, i.e. typically 0.5-1.0 mm, and so do the derived conductivity/permittivity images. Moreover, both the electric conductivity and the permittivity can be derived within the current approach in opposite to "noise tomography" [3], where only the electric conductivity can be derived.

Furthermore, the proposed invention provides the possibility to estimate the local SAR via

$$SAR_{local} = \int\limits_{\substack{local \\ region}} \sigma(\vec{r})\underline{E}(\vec{r})\underline{E}^{\bullet}(\vec{r})dV \ , \tag{0}$$

which would provide a more accurate SAR measure than the global SAR estimations used up to now. Also via MR-temperature measurements a thermal model including thermal capacity and conductivity of the patient can be generated.

From Maxwell's equations, the following equation containing a complex permittivity can be derived

$$\nabla \times \underline{\vec{H}}(\vec{r}) = i\omega\underline{\varepsilon}(\vec{r})\vec{E}(\vec{r}) \tag{1}$$

with $\underline{H}$ the magnetic field strength, $\underline{E}$ the electric field, $\omega$ the Larmor frequency, and $\underline{\varepsilon}$ the permittivity. The underlines denote complex variables. Eq. (1) can be solved for the unknown $\underline{\varepsilon}$ by regarding only the z-component

$$\left(\partial_x\underline{H}_y(\vec{r}) - \partial_y\underline{H}_x(\vec{r})\right)/\underline{E}_z(\vec{r}) = i\omega\underline{\varepsilon}(\vec{r}) \ . \tag{2}$$

Here, $\underline{H}_x$ and $\underline{H}_y$ can be measured via MRI, and $\underline{E}_z$ has to be estimated via simulations. The real and imaginary part of $\underline{\varepsilon}$ can be identified with the (non-complex) permittivity $\varepsilon$ and the electric conductivity $\sigma$, respectively.

**Central equation**

We start from Maxwell's extension of the Ampere law,

$$\nabla \times \vec{H}(\vec{r},t) = \vec{j} + \partial_t\vec{D}(\vec{r},t), \tag{3}$$

with $j$ denoting the current density and D the electric displacement field. Using
$\vec{D} = \varepsilon\vec{E}$
and
$\vec{j} = \sigma\vec{E},$
Eq. (3) can be rewritten as

$$\nabla \times \vec{H}(\vec{r},t) = \sigma(\vec{r})\vec{E}(\vec{r},t) + \varepsilon(\vec{r})\partial_t\vec{E}(\vec{r},t) \tag{4}$$

assuming $\varepsilon$ constant in time. Further assuming
$\vec{E}(r,t) =: real\{\vec{E}(r)\exp(i\omega t)\}, \ \vec{H}(r,t) =: real\{\vec{H}(r)\exp(i\omega t)\},$
Eq. (4) reads as given in Eq. (1)

$$\nabla \times \underline{\vec{H}}(\vec{r}) = (\sigma(\vec{r}) + i\omega\varepsilon(\vec{r}))\underline{\vec{E}}(\vec{r}) = i\omega\underline{\varepsilon}(\vec{r})\underline{\vec{E}}(\vec{r}) \ , \qquad (1')$$

introducing the complex permittivity

$$\underline{\varepsilon}(\vec{r}) \equiv \varepsilon(\vec{r}) - i\sigma(\vec{r})/\omega \ . \qquad (5)$$

The real and imaginary parts of the right hand side of Eq.(1') are sometimes called "eddy current" and "displacement current", respectively. As mentioned above, Eq. (1) can be solved for the unknown $\underline{\varepsilon}$ by regarding the z-component

$$\left(\partial_x \underline{H}_y(\vec{r}) - \partial_y \underline{H}_x(\vec{r})\right) / \underline{E}_z(\vec{r}) = i\omega\underline{\varepsilon}(\vec{r}) \ . \qquad (2')$$

In principle, Eq. (2) can also be formulated for the x- or y-component, however, the occurring $\underline{H}_z$ cannot be measured within MRI (assuming $B_0$ in z-direction).

Using N coils producing the fields $H^n$ and $E^n$ ($n = 1...N$), Eq. (2') can be replaced by

$$\left(\partial_x \underline{H}_y^{tot}(\vec{r}) - \partial_y \underline{H}_x^{tot}(\vec{r})\right) / \underline{E}_z^{tot}(\vec{r}) = i\omega\underline{\varepsilon}(\vec{r}) \qquad (2'')$$

with

$$\underline{\vec{H}}^{tot} = \sum_{n \leq N} \underline{\vec{H}}^n \quad ; \quad \underline{\vec{E}}^{tot} = \sum_{n \leq N} \underline{\vec{E}}^n \ .$$

Using $N>1$ coils, it is easier to avoid regions with $E^{tot}$ close to zero, which would lead to noise amplification in the resulting images. In other words, for every pixel there should be at least one coil producing a significant $E$ field, avoiding the division by small numbers. Also more than one combination of coils can be used to measure at least twice and solve simultaneously the same conductivity and permittivity distribution.

**Determination of H field**

The spatial transmit sensitivity distribution of an RF coil is given by the $H$ component circularly polarized in one direction (which might be defined as the "positive" direction) [4] If the static magnetic field has a negative z-direction, the transmit component is given by

$$\underline{H}^+ = \left(\underline{H}_x + i\underline{H}_y\right)/2 \ . \qquad (6)$$

The spatial *receive* sensitivity distribution of an RF coil is given by the $H$ component circularly polarized in the opposite direction than in the transmit case (i.e. the "negative" direction) [4]

$$\underline{H}^- = \left(\underline{H}_x - i\underline{H}_y\right)^*/2 \ . \qquad (7)$$

Thus, the wanted components $\underline{H}_x$ and $\underline{H}_y$ can be deduced from Eqs. (6,7). Alternatively, the numerator in Eq. (2) might be replaced by

$$\partial_x \underline{H}_y(\vec{r}) - \partial_y \underline{H}_x(\vec{r}) = i\partial_x \left( \underline{H}^{-*}(\vec{r}) - \underline{H}^{+}(\vec{r}) \right) - \partial_y \left( \underline{H}^{-*}(\vec{r}) + \underline{H}^{+}(\vec{r}) \right) . (8)$$

The transmit and receive sensitivities can be determined, e.g., using the relation [4]

$$\underline{S}(\vec{r}) \sim \underline{M}_o(\vec{r}) \underline{H}^{-}(\vec{r}) \sin( const \cdot \underline{H}^{+}(\vec{r})) . \qquad (9)$$

Here, $\underline{S}$ is the image signal intensity and *const* is a system dependent constant. Thus, $\underline{H}^{+}$ and $\underline{H}^{-}$ can be obtained by acquiring a series of images with different flip angles and fitting a sinus in each pixel according to Eq. (9).

Using a quadrature body coil, it might be a good approximation to set $\underline{H}^{-}$ to zero, since this coil is designed to produce only one direction of the circularly polarized field.

An alternative method is to rotate the patient, i.e., to measure him a second time placing feet first instead head first. Ideally, also the coil is rotated, which should be possible in case of using a head or surface coil.

It would also be sufficient to switch the main field, which is, of course, out of the scope for today's high field systems, but might be realized with a low field system.

**Determination of (raw) E field**

First, the raw E field $\underline{E}_o$ has to be determined in the framework of a numerical simulation. The input for this simulation is (a) the geometry of the used RF coil(s), (b) the feeding voltage(s) of the used RF coil(s), (c) the geometry of the patient's body with respect to the position of the RF coil(s), taken from the images acquired for the H field determination, (d) a mean $\varepsilon$ throughout the patient's body or literature values of $\varepsilon$ for segmented body compartments. The simulation can be performed using numerical electromagnetic field calculations, e.g., a finite difference time domain algorithm or the method of moments. The correctness of the model can be checked by comparing measured and simulated H field components.

**Iterative solution**

With the help of the raw E°field $\underline{E}_o$, a first estimation of the permittivity $\varepsilon_1$ is obtained. Then, an improved E field $\underline{E}_1$ can be derived replacing $\varepsilon_o$ by $\varepsilon_1$ in the simulation framework, leading to a refined patient model. With the help of $\underline{E}_1$, an improved permittivity estimation $\varepsilon_2$ is obtained, and so on (see Fig. 1). The iteration might be stopped using suitable convergence criteria, e.g., if $|\varepsilon_i - \varepsilon_{i-1}| < \delta$.

**Comparison with Noise Tomography**

In the presented approach, the obtained spatial resolution should be of the order of the spatial resolution of the measured H field components. This seems to be the main advantage compared with noise tomography [3], where the spatial resolution is limited by the smoothness of the spatial E field distribution. Another advantage of the current approach is the possibility to directly derive the permittivity of the tissue. A third difference is given by the required mathematics. Noise tomography defines an inverse problem, which can be solved, e.g., via matrix inversion or back-projection. The presented approach replaces the inverse problem by a differentiation, which is also a non-trivial task. However, there is hope that this differentiation might be easier to handle than the inverse problem of the noise tomography.

**Further remarks**

In principle, Eq. (2) is applicable for a single slice. However, for the simulation of the E field, a 3D volume is required. Thus, a 3D acquisition of the H field is preferred to support the E field model.

In regions of low E field, Eq. (2) is prone to errors due to dividing by small numbers. This problem might be circumvented by the use of multiple RF coils with (spatially) complementary E fields. Additionally, the patient might be shifted relative to the coils (or the coils might be shifted relative to the patient) to increase the amount of acquired data and enhance the image reconstruction stability.

If only one component $\underline{H}^{+}$ or $\underline{H}^{-}$ is known, the missing component might also be taken from the E field simulation. Thus, it is not necessary to use a transmit/receive coil, but transmitting with the QBC and analysing data from a (receive-only) surface coil would also be sufficient. In an ideal situation, the results should be the same as measuring both H field components. In a real experiment, the reduced amount of experimental input data will lead to an image quality reduction.

In Eq. (1), $\varepsilon$ and $\sigma$ are assumed to be scalar, i.e., isotropic. In general, $\varepsilon$ and $\sigma$ can be tensors, i.e., $\varepsilon$ and $\sigma$ might differ for different spatial directions. Different directions of $\varepsilon$ and $\sigma$ can be measured rotating the Bo field relative to the patient. This can be done, e.g., by tilting the patient on the side, or by measuring the patient in different MR systems, e.g., a standard cylindrical system with Bo oriented parallel to the patient's longitudinal axis, and an open MR system with Bo oriented perpendicular to the patient's longitudinal axis.

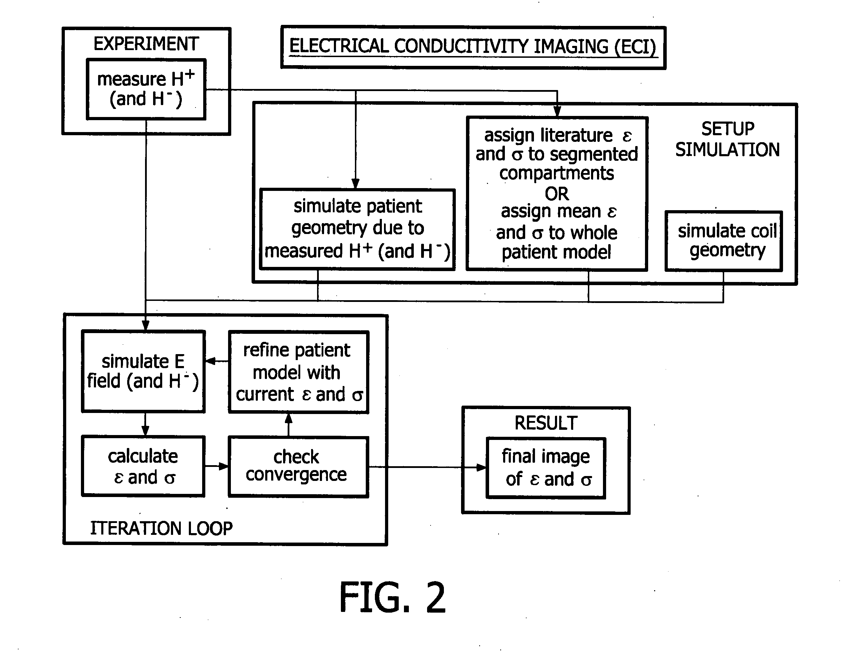

The basic numerical feasibility of the approach was demonstrated (see Fig. 2). The electromagnetic fields in ($\varepsilon$=80, $\sigma$=0.5S/m) and around ($\varepsilon$=1, $\sigma$=0) a homogeneous sphere have been calculated for a single loop coil on the left hand side of the FOV to simulate the measurement of the H field and the modelling of the E field. A tumour model has been inserted with $\varepsilon$=80, $\sigma$=0.6S/m. Then, the data have been analysed using Eq. (2) reproducing the input values of $\sigma$ (left) and $\varepsilon$ (right). Five iterations according to Fig. 1 have been performed, starting with E fields from a homogeneous sphere without tumour model. Noise has been added to the simulated measurement of the H fields. The noise in the centre is caused by E fields close to zero. To avoid this noise, a second measurement with a coil located, e.g., above or below the FOV should be performed.

Electric conductivity imaging might result in a new modality of medical imaging and many applications can be imagined. For instance, it could be used to distinguish between tumours and healthy tissue [5] or between necrotic and vital tissue after a myocardial infarction. It might also be used to support the characterization of brain tissue in connection with stroke or cerebral haemorrhage. Another application might be given by improved RF ablation planning. The method might also be applied in non-medical areas, e.g., contact-less material testing, as long as the material is suitable for MR.

References

| [1] | Gabriel S, Lau RW, Gabriel C. The dielectric properties of biological tissues: II. Measurements in the frequency range 10 Hz to 20 GHz. Phys Med Biol. 1996 Nov;41(11):2251-69. |
|---|---|
| [2] | Fuks LF, Cheney M, Isaacson D, Gisser DG, Newell JC. Detection and imaging of electric conductivity and permittivity at low frequency. IEEE Trans Biomed Eng. 1991 Nov;38(11):1106-10. |
| [3] | Duensing GR, Saylor C, Huang F, Method and apparatus for noise tomography, pat. US2003/0160622 |
| [4] | Collins CM, Yang QX, Wang JH, Zhang X, Liu H, Michaeli S, Zhu XH, Adriany G, Vaughan JT, Anderson P, Merkle H, Ugurbil K, Smith MB, Chen W. Different excitation and reception distributions with a single-loop transmit-receive surface coil near a head-sized spherical phantom at 300 MHz. Magn Reson Med. 2002 May;47(5):1026-8 |
| [5] | Haemmerich D, Staelin ST, Tsai JZ, Tungjitkusolmun S, Mahvi DM, Webster JG. In vivo electrical conductivity of hepatic tumours. Physiol Meas. 2003 May;24(2):251-60. |

**Claims**

1. An electric impedance imaging system to explore an object's electrical permittivity distribution, the electric impedance imaging system including a set of main coils to generate a steady uniform magnetic field and the electrical impedance imaging system having means for:

   - application of an excitation electro-magnetic field to excite spins of the object;
   - acquisition of magnetic resonance signals from the object and generated due the excitation electro-magnetic field;
   - computation of an electric field strength distribution associated with the excitation electro-magnetic field;
   - derivation of a magnetic induction field strength distribution from the acquired magnetic resonance signals and **characterised in that** the electric impedance imaging system has further means for:
   - computation of the electrical permittivity and conductivity distribution, at the Larmor frequency that corresponds to the steady uniform magnetic field from the electric field strength distribution and the magnetic induction field strength distribution.

2. An electric impedance imaging system as claimed in Claim 1 wherein

   - application of the excitation electro-magnetic field involves a circular polarised magnetic induction field with an excitation polarisation orientation
- acquisition of the magnetic resonance signals involves a circular polarised magnetic induction field with a reception polarisation orientation.

3. An electric impedance imaging system as claimed in Claim 2, having the means for:

- application of the excitation electro-magnetic field for several values of a flip angle of the excited spins.

4. An electric impedance imaging system as claimed in Claim 1 having means for iteratively

- estimating the electric field strength distribution
- computing an electrical permittivity from the estimated electric field strength distribution
- computing an update electric field strength distribution from the computed electrical permittivity distribution and
- employing the update electric field strength distribution as a new estimate of the electric field strength distribution.

5. An electric impedance imaging system as claimed in Claim 3, wherein at least one of the reception polarisation orientated or excitation polarisation orientated magnetic induction field component is derived from the electric field strength distribution.

6. An electric impedance imaging system as claimed in Claim 1 comprising an RF transmit antenna to emit the excitation electro-magnetic field and which has a switchable polarisation orientation sensitivity.

7. An electric impedance imaging system as claimed in Claim 1, comprising

- several RF receive antennae and
- individual RF receive antennae having complementary spatial sensitivity profiles for the electrical field strength distribution.

8. An electric impedance imaging system as claimed in Claim 1, having means for reconstructing an image data set from the electrical permittivity distribution, in particular in the form of a volumetric data set or in the form of a tomographic image.

9. An electric impedance imaging method to explore an object's electrical permittivity distribution, the electric impedance imaging method including the steps of

- application of a steady uniform magnetic field by way of a set of main coils,
- application of an excitation electro-magnetic field to excite spins of the object

  - acquisition of magnetic resonance signals from the object and generated due the excitation electro-magnetic field
  - computation of an electric field strength distribution associated with the excitation electro-magnetic field
  - derivation of a magnetic induction field strength distribution from the acquired magnetic resonance signals and
  - computation of the electrical permittivity and conductivity distribution at the Larmor frequency that corresponds to the steady uniform magnetic field from the electric field strength distribution and the magnetic induction field strength distribution.

10. A computer program to explore an object's electrical permittivity distribution, the computer program including instructions which when executed on a system according to Claim 1 causes application of an excitation electro-magnetic field to excite spins of the object and

- acquisition of magnetic resonance signals from the object and generated due the excitation electro-magnetic field;
- computation of an electric field strength distribution associated with the excitation electro-magnetic field j
- derivation of a magnetic induction field strength distribution from the acquired magnetic resonance signals; and
- computation of the dynamic electrical permittivity and dynamic conductivity distribution, at the Larmor frequency that corresponds to an steady uniform magnetic field applied by way of a set of main coils, from the electric field strength distribution and the magnetic induction field strength distribution.

**Patentansprüche**

1. Elektroimpedanz-Bildgebungssystem zur Untersuchung der Verteilung der elektrischen Permittivität eines Objekts, wobei das Elektroimpedanz-Bildgebungssystem einen Satz Hauptspulen umfasst, um ein konstantes homogenes Magnetfeld zu erzeugen und wobei das Elektroimpedanz-Bildgebungssystem Mittel umfasst zum

   - Anlegen eines elektromagnetischen Anregungsfeldes, um Spins des Objekts anzuregen;
   - Erfassen von Magnetresonanzsignalen von dem Objekt, die aufgrund des elektromagnetischen Anregungsfeldes erzeugt wurden;
   - Berechnen einer zu dem elektromagnetischen Anregungsfeld gehörigen Verteilung der elektrischen Feldstärke;
   - Ableiten einer Verteilung der magnetischen Induktionsfeldstärke von den erfassten Magnetresonanzsignalen und **dadurch gekennzeichnet, dass** das Elektroimpedanz-Bildgebungssystem weiterhin Mittel umfasst zum
   - Berechnen der Verteilung der elektrischen Permittivität und Leitfähigkeit bei der dem konstanten homogenen Magnetfeld entsprechenden Larmor-Frequenz anhand der Verteilung der elektrischen Feldstärke und der Verteilung der magnetischen Induktionsfeldstärke.

2. Elektroimpedanz-Bildgebungssystem nach Anspruch 1, wobei

   - das Anlegen eines elektromagnetischen Anregungsfeldes ein kreisförmig polarisiertes magnetisches Induktionsfeld mit einer Anregungspolarisationsorientierung beinhaltet;
   - das Erfassen der Magnetresonanzsignale ein kreisförmig polarisiertes magnetisches Induktionsfeld mit einer Empfangspolarisationsorientierung beinhaltet.

3. Elektroimpedanz-Bildgebungssystem nach Anspruch 2 mit Mitteln zum

   - Anlegen des elektromagnetischen Anregungsfeldes für mehrere Werte eines Flipwinkels der angeregten Spins.

4. Elektroimpedanz-Bildgebungssystem nach Anspruch 1 mit Mitteln zum iterativen

   - Schätzen der Verteilung der elektrischen Feldstärke,
   Berechnen einer elektrischen Permittivität anhand der geschätzten Verteilung der elektrischen Feldstärke,
   - Berechnen einer aktualisierten Verteilung der elektrischen Feldstärke anhand der berechneten Verteilung der elektrischen Permittivität und
   - Verwenden der aktualisierten Verteilung der elektrischen Feldstärke als einen neuen Schätzwert der Verteilung der elektrischen Feldstärke.

5. Elektroimpedanz-Bildgebungssystem nach Anspruch 3, wobei mindestens entweder die empfangspolarisationsorientierte oder die anregungspolarisationsorientierte magnetische Induktionsfeldkomponente von der Verteilung der elektrischen Feldstärke abgeleitet wird.

6. Elektroimpedanz-Bildgebungssystem nach Anspruch 1 mit einer HF-Sendeantenne zum Emittieren des elektromagnetischen Anregungsfeldes und die eine umschaltbare Polarisationsorientierungsempfindlichkeit hat.

7. Elektroimpedanz-Bildgebungssystem nach Anspruch 1 mit

   - mehreren HF-Empfangsantennen und
   - wobei einzelne HF-Empfangsantennen komplementäre räumliche Empfindlichkeitsprofile für die Verteilung der elektrischen Feldstärke haben.

8. Elektroimpedanz-Bildgebungssystem nach Anspruch 1 mit Mitteln zum Rekonstruieren eines Bilddatensatzes anhand der Verteilung der elektrischen Permittivität, insbesondere in der Form eines volumetrischen Datensatzes oder in der Form eines tomographischen Bildes.

9. Elektroimpedanz-Bildgebungsverfahren zur Untersuchung der Verteilung der elektrischen Permittivität eines Objekts, wobei das Elektroimpedanz-Bildgebungsverfahren die folgenden Schritte umfasst:

- Anlegen eines konstanten homogenen Magnetfelds mit Hilfe eines Satzes Hauptspulen;
- Anlegen eines elektromagnetischen Anregungsfeldes, um Spins des Objekts anzuregen;
- Erfassen von Magnetresonanzsignalen von dem Objekt, die aufgrund des elektromagnetischen Anregungsfeldes erzeugt wurden;
- Berechnen einer zu dem elektromagnetischen Anregungsfeld gehörigen Verteilung der elektrischen Feldstärke;
- Ableiten einer Verteilung der magnetischen Induktionsfeldstärke von den erfassten Magnetresonanzsignalen und
- Berechnen der Verteilung der elektrischen Permittivität und Leitfähigkeit bei der dem konstanten homogenen Magnetfeld entsprechenden Larmor-Frequenz anhand der Verteilung der elektrischen Feldstärke und der Verteilung der magnetischen Induktionsfeldstärke.

10. Computerprogramm zur Untersuchung der Verteilung der elektrischen Permittivität eines Objekts, wobei das Computerprogramm Anweisungen umfasst, die bei Ausführung auf einem System nach Anspruch 1 Folgendes bewirken:

- Anlegen eines elektromagnetischen Anregungsfeldes, um Spins des Objekts anzuregen;
- Erfassen von Magnetresonanzsignalen von dem Objekt, die aufgrund des elektromagnetischen Anregungsfeldes erzeugt wurden;
- Berechnen einer zu dem elektromagnetischen Anregungsfeld gehörigen Verteilung der elektrischen Feldstärke;
- Ableiten einer Verteilung der magnetischen Induktionsfeldstärke von den erfassten Magnetresonanzsignalen und
- Berechnen der Verteilung der elektrischen Permittivität und Leitfähigkeit bei der Lamor-Frequenz, die dem mit Hilfe eines Satzes Hauptspulen angelegten konstanten homogenen Magnetfeld entspricht, anhand der Verteilung der elektrischen Feldstärke und der Verteilung der magnetischen Induktionsfeldstärke.

## Revendications

1. Système d'imagerie à impédance électrique pour explorer la distribution de permittivité électrique d'un objet, le système d'imagerie à impédance électrique comprenant un ensemble de bobines principales pour générer un champ magnétique uniforme constant et le système d'imagerie à impédance électrique ayant des moyens pour effectuer :

- l'application d'un champ électromagnétique d'excitation pour exciter des spins de l'objet ;
- l'acquisition de signaux de résonance magnétique de l'objet générés en raison du champ électromagnétique d' excitation ;
- le calcul d'une distribution de force de champ électrique associée au champ électromagnétique d' excitation ;
- la dérivation d'une distribution de force de champ d'induction magnétique à partir des signaux de résonance magnétique acquis ; et **caractérisé en ce que** le système d'imagerie à impédance électrique comprend en outre des moyens pour effectuer :
- le calcul de la distribution de permittivité et de conductivité électrique à la fréquence de Larmor correspondant au champ magnétique uniforme constant à partir de la distribution de force de champ électrique et de la distribution de force de champ d'induction magnétique.

2. Système d'imagerie à impédance électrique selon la revendication 1, dans lequel

- l'application du champ électromagnétique d'excitation implique un champ d'induction magnétique polarisé circulaire avec une orientation de polarisation d'excitation ;
- l'acquisition des signaux de résonance magnétique implique un champ d'induction magnétique polarisé circulaire avec une orientation de polarisation de réception.

3. Système d'imagerie à impédance électrique selon la revendication 2, comportant des moyens pour effectuer :

- l'application du champ électromagnétique d'excitation pour plusieurs valeurs d'un angle de bascule des spins excités.

**4.** Système d'imagerie à impédance électrique selon la revendication 1, comportant des moyens pour effectuer itérativement :

- l'estimation de la distribution de force de champ électrique ;
- le calcul d'une permittivité électrique à partir de la distribution de force de champ électrique estimée ;
- le calcul d'une distribution de force de champ électrique actualisée à partir de la distribution de permittivité électrique calculée ; et
- l'emploi de la distribution de force de champ électrique actualisée en tant que nouvelle estimation de la distribution de force de champ électrique.

**5.** Système d'imagerie à impédance électrique selon la revendication 3, dans lequel au moins l'un du composant de champ d'induction magnétique orienté par polarisation à réception et du composant de champ d'induction magnétique orienté par polarisation à excitation est dérivé de la distribution de force de champ électrique.

**6.** Système d'imagerie à impédance électrique selon la revendication 1, comprenant une antenne de transmission RF pour émettre le champ électromagnétique d'excitation et qui a une sensibilité d'orientation de polarisation commutable.

**7.** Système d'imagerie à impédance électrique selon la revendication 1, comprenant

- plusieurs antennes de réception RF ; et
- des antennes de réception RF individuelles ayant des profils de sensibilité spatiale complémentaires pour la distribution de force de champ électrique.

**8.** Système d'imagerie à impédance électrique selon la revendication 1, comportant des moyens pour reconstruire un ensemble de données d'image à partir de la distribution de permittivité électrique, en particulier sous la forme d'un ensemble de données volumétriques ou sous la forme d'une image tomographique.

**9.** Procédé d'imagerie à impédance électrique pour explorer la distribution de permittivité électrique d'un objet, le procédé d'imagerie à impédance électrique comprenant les étapes de :

- l'application d'un champ magnétique uniforme constant au moyen d'un ensemble de bobines principales ;
- l'application d'un champ électromagnétique d'excitation pour exciter des spins de l'objet ;
- l'acquisition de signaux de résonance magnétique de l'objet générés en raison du champ électromagnétique d'excitation ;
- le calcul d'une distribution de force de champ électrique associée au champ électromagnétique d'excitation ;
- la dérivation d'une distribution de force de champ d'induction magnétique à partir des signaux de résonance magnétique acquis ; et
- le calcul de la distribution de permittivité et de conductivité électrique à la fréquence de Larmor correspondant au champ magnétique uniforme constant à partir de la distribution de force de champ électrique et de la distribution de force de champ d'induction magnétique.

**10.** Programme d'ordinateur pour explorer la distribution de permittivité électrique d'un objet, le programme d'ordinateur comprenant des instructions qui, lorsqu'elles sont exécutées sur un système selon la revendication 1, provoquent :

- l'application d'un champ électromagnétique d'excitation pour exciter des spins de l'objet ; et
- l'acquisition de signaux de résonance magnétique de l'objet générés en raison du champ électromagnétique d' excitation ;
- le calcul d'une distribution de force de champ électrique associée au champ électromagnétique d' excitation ;
- la dérivation d'une distribution de force de champ d'induction magnétique à partir des signaux de résonance magnétique acquis ; et
- le calcul de la distribution de permittivité électrique dynamique et de conductivité dynamique à la fréquence de Larmor correspondant à un champ magnétique uniforme constant appliqué au moyen d'un ensemble de bobines principales, à partir de la distribution de force de champ électrique et de la distribution de force de champ d'induction magnétique.

FIG. 1

FIG. 2

FIG. 3a

FIG. 3b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

•   US 20030160622 A

### Non-patent literature cited in the description

•   **L.T. MUFTULER.** Resolution and constast in magnetic resonance electrical impedance tomography (MREIT) and its application to cancer imaging. *Technol. cancer Res. Treat,* 2004, vol. 3, 599-609
•   Electromagnetic impedance tomography (EMIT): a new method form impedance imaging. *IEEE Trasn.Med.Imaging,* 2002, vol. 21, 676-687
•   **GABRIEL S ; LAU RW ; GABRIEL C.** The dielectric properties of biological tissues: II. Measurements in the frequency range 10 Hz to 20 GHz. *Phys Med Biol.,* November 1996, vol. 41 (11), 2251-69
•   **FUKS LF ; CHENEY M ; ISAACSON D ; GISSER DG ; NEWELL JC.** Detection and imaging of electric conductivity and permittivity at low frequency. *IEEE Trans Biomed Eng.,* November 1991, vol. 38 (11), 1106-10

•   **COLLINS CM ; YANG QX ; WANG JH ; ZHANG X ; LIU H ; MICHAELI S ; ZHU XH ; ADRIANY G ; VAUGHAN JT ; ANDERSON P.** Different excitation and reception distributions with a single-loop transmit-receive surface coil near a head-sized spherical phantom at 300 MHz. *Magn Reson Med.,* May 2002, vol. 47 (5), 1026-8
•   **HAEMMERICH D ; STAELIN ST ; TSAI JZ ; TUNGJITKUSOLMUN S ; MAHVI DM ; WEBSTER JG.** In vivo electrical conductivity of hepatic tumours. *Physiol Meas,* May 2003, vol. 24 (2), 251-60